# EUROPÄISCHE PATENTANMELDUNG

(11) **EP 4 145 105 A1**
(43) Veröffentlichungstag der Anmeldung: **08.03.2023**
(21) Anmeldenummer: 21195103.3
(22) Anmeldetag: 06.09.2021
(51) Int. Cl.: G01N 1/30, C12N 5/09

(54) **LÖSUNG FÜR DIE ASSERVIERUNG VON ZELLEN**

(71) Anmelder: Anacyte Laboratories GmbH, 22393 Hamburg (DE)
(72) Erfinder: Die Erfindernennung liegt noch nicht vor
(74) Vertreter: Böhm, Brigitte

(57) **Zusammenfassung**

Die Erfindung betrifft eine Lösung für die Asservierung von Zellen, insbesondere von Tumorzellen in Blutproben oder anderen Körperflüssigkeiten. Die Lösung enthält einen zellmembrangängigen Protonencarrier und ist frei von chaotropen Substanzen. Eine solche Lösung wird vorteilhaft in Proberöhrchen, Blutentnahmespritzen oder Blutentnahmeröhrchen eingesetzt.

## Beschreibung

Die vorliegende Erfindung betrifft generell die Stabilisierung von Blutproben und insbesondere die Asservierung von Zellen, vor allem von zirkulierenden Tumorzellen, in Blutproben oder anderen Körperflüssigkeiten. Ferner betrifft die Erfindung die Verwendung einer solchen asservierenden Lösungen sowie Proberöhrchen oder Blutentnahmeröhrchen, in denen eine erfindungsgemäße Lösung vorgelegt ist.

### Hintergrund der Erfindung

In der Tumorbiologie konnte in den letzten Jahren eine große Heterogenität der molekularen Ausstattung von Zellen innerhalb eines Tumors nachgewiesen werden. In diesem Zusammenhang wird zirkulierenden Tumorzellen (CTC) besondere Bedeutung zugeschrieben, da eine Analyse dieser Zellen ermöglicht, Therapieoptionen aufzuzeigen sowie den Krankheit- und Therapieverlauf zu überwachen. Zirkulierende Tumorzellen sind Zellen, die sich von einem Primärtumor abgelöst haben. Sie können entweder im Lymphsystem vorliegen oder aber im Blutkreislauf zirkulieren. Zumindest bei einigen Arten von zirkulierenden Tumorzellen besteht die Möglichkeit, dass sie sich in anderen Organen oder Geweben ansiedeln und neue Tumore bzw. Metastasen bilden. Die Mehrheit der krebsbedingten Todesfälle ist nicht auf Primärtumore zurückzuführen, sondern auf davon abgeleitete Metastasen und Sekundärtumore.

Diagnostische Methoden, welche es erlauben, CTCs zu analysieren, können wesentlich zur Auswahl von personalisierten und effektiven Behandlungsmethoden beitragen. Ferner, kann die Analyse von CTCs bei der Erstellung einer Prognose für den Patienten in einem frühen Stadium hilfreich sein, da man mittlerweile weiß, dass diese zirkulierenden Zellen bereits in einem frühen Krankheitsstadium existieren und nachgewiesen werden können. Auch für die Untersuchung der Wirksamkeit von Medikamenten sind Analysen von CTCs von hoher Bedeutung.

CTCs liegen allerdings nur in äußerst geringen Mengen in den jeweiligen Körperflüssigkeiten vor und sind darüber hinaus empfindlich gegenüber Manipulationen im Rahmen der Analysen. Alle Biomoleküle unterliegen in einer intakten, stoffwechselnden Zelle außerdem einem ständigen Umsatz. Abhängig von unterschiedlichen Einflussfaktoren, zum Beispiel Umweltbedingungen oder Differenzierungsstadium oder einer anderen, den Metabolismus beeinflussenden Gegebenheit, erfolgt dieser Umsatz von Molekülen durch Neusynthese und Abbau. Eine Änderung der biomolekularen Ausstattung geschieht innerhalb von Zellen spezifisch, kontrolliert und zielgerichtet. Zum Verständnis biologischer Funktionen eines Moleküls in einer Zelle ist es von entscheidender Bedeutung, die jeweilige Funktion im Kontext der gleichzeitig anwesenden weiteren Biomoleküle zu beobachten. Hierzu ist eine Konservierung von Zellen und eine Stabilisierung ihres metabolischen und morphologischen Zustands erforderlich.

Obwohl die Existenz von CTCs grundsätzlich seit langem bekannt ist, rückten sie erst vor einigen Jahren in das Interesse der Forschung, um die Behandlungsmöglichkeiten von Tumorpatienten zu verbessern. Für die Konservierung von Blut sind einige Wege seit Jahrzehnten weltweiter Standard. EDTA-, Citrat- oder Heparin-Zusätze verhindern die Blutgerinnung und erlauben es die Bestandteile des Blutes zu analysieren. CTC können mit Hilfe dieser Zusätze allerdings nicht sehr gut stabilisiert werden, da viele normale Blutfunktionen auch nach der Blutentnahme, also ex *vivo,* erhalten bleiben. So reagiert beispielsweise das Immunsystem und versucht, CTCs zu eliminieren, oder von einer Chemotherapie bereits angegriffene CTCs unterliegen Nekrose oder Apoptose.

Die Lagerungszeit zwischen der Entnahme und der Weiterverarbeitung von Blutproben kann außerdem viele Stunden betragen und birgt erhebliche Risiken. Der Expressionsstatus der CTCs ändert sich mit Temperatur und Umgebungsbedingungen, auf welche die CTC mit geänderter Genexpression reagieren. Dies führt zu einer unerwünschten Änderung des molekularen Abbilds in der nachfolgenden Analyse.

Es ist ferner bekannt, dass auch normale Blutzellen erheblicher Veränderung durch Lagerung unterliegen. Auf der anderen Seite gehört die Lagerung von Blut zur diagnostischen Routine, da im Krankenhausalltag bestimmte Abläufe und Zeiten vorgegeben sind. Zu den lagerungsbedingten Veränderungen ist nicht nur eine Änderung des Expressionsstatus von Blutzellen zu zählen, sondern auch bspw. eine Art Desintegration von Zellen, was sogenannten Debris induziert, also Aggregate herumliegenden Zellmaterials, die nicht nur die Analyse von Zellen erschweren, sondern auch invalide Analysen verursachen können (siehe hierzu auch US 7,863,012 B2). Hierdurch wird ersichtlich, dass nicht nur der Erhalt der zellulären Integrität, sondern auch der Erhalt des Expressionsstatus von Zellen über einen längeren Zeitraum von entscheidender Bedeutung für die Analyse von CTCs ist. Insbesondere ist die RNA-Stabilität von Bedeutung, da durch das Transkriptom entscheidende Erkenntnisse zu Tumorbiologie und therapeutischen Optionen gewonnen werden können.

### Stand der Technik

In der Literatur wurden verschiedene Wege vorgeschlagen, wie diese zelluläre Integrität aufrechterhalten werden kann. In der Regel basieren diese auf vernetzenden Agenzien wie Formaldehyd, Paraformaldehyd, Glutaraldehyd (bspw. EP 0 214 613 A2, DE 40 39 716 A1). Ein weiterer Ansatz wird durch Offenlegungen aufgezeigt, in denen Methylol-Derivate eine Fixierung der Blutbestandteile ermöglichen. Methylol wird auch als Konservierungsmittel in der Kosmetikindustrie verwendet. Der exakte Wirkmechanismus ist unbekannt, Formaldehyd scheint jedoch eine bedeutende Rolle hierbei zu spielen. Die quervernetzende Natur dieser Fixierungslösungen, insbesondere auch die Quervernetzung von Nukleinsäuren, erschwert aber die Analyse von Zellen auf molekularer Ebene. Dieses Problem versucht unter anderem die US 5976829 A durch ein Fixativ zu lösen, welches einen Aldehyd, einen Alkohol, einen Chelatbildner und einen keine Aminogruppen enthaltenden Puffer umfasst.

Im wissenschaftlichen Bereich werden für die Fixierung von Zellen bzw. der Moleküle auch weitere Methoden verwendet. Hierzu gehören bspw. das Einfrieren bei tiefen Temperaturen, Einsatz von alkoholhaltigen Fixativen (wie z.B. Methanol, Ethanol, Glycol), oder auch von chaotropen Reagenzien (wie z.B. Isothiocyanat), welche für eine Stabilisierung von Molekülen unter Zerstörung morphologischer Zusammenhänge auch kommerziell angeboten werden (z. B. RNAlater, ThermoFisher; AllProtect, Qiagen).

Im Stand der Technik sind außerdem verschiedene Lösungen verfügbar, um Gewebe ohne Quervernetzung bei gleichzeitigem Strukturerhalt zu fixieren. EP 2 126 542 B1, FR 2 852 392 A1, WO 2013/131816 A1 und WO 03/029783 A1 beschreiben Gemische zur Gewebefixierung unter Verwendung von organischen Lösungsmitteln. Eine Abwandlung ohne organisches Lösungsmittel, aber unter Einsatz von Bisulfiten ist in US 5,432,056 A beschrieben. Hier wird ein Bisulfit-haltiges saures System vorgeschlagen, um Gewebeschnitte oder andere dünnschichtige Proben zu fixieren. US 6,337,189 B1 wiederum beschreibt eine Harnstoffverbindung in Verbindung mit Alkohol zur nichtvernetzenden Fixierung zytologische Präparate. Der morphologische Erhalt zellulärer Strukturen ist allerdings bei all diesen Methoden beschränkt.

Für eine Konservierung von Vollblut für die Analyse von CTCs sind solche Methoden in der Regel ungeeignet, da sie entweder nicht für die Routinediagnostik geeignet sind, die zelluläre Integrität zerstören oder keine effektive Molekülstabilisierung gewährleisten.

US10,091,984 B2 lehrt allerdings, dass eine Formaldehyd-freisetzende Harnstoffverbindung, bei gleichzeitigem Abfangen des Formaldehyds durch Glycin geeignet ist, CTCs morphologisch zu stabilisieren. Auch wenn diese Offenlegung keine kovalente Modifikation der CTC Moleküle postuliert, ist die Rolle des Formaldehyds unklar.

Die zu lösende Aufgabe bestand daher darin, die Stabilisierung von Vollblut, insbesondere von CTCs, über einen in der Routinediagnostik üblichen Zeitraum in solcher Weise zu ermöglichen, dass ex *vivo* Lagerungsartefakte vermieden werden, und die CTCs sowohl morphologisch als auch molekular, also im Hinblick auf ihre zellulären Biomoleküle, ohne Quervernetzung stabilisiert werden und so Genetik und Expressionsstatus der CTCs erhalten bleiben, um sie effektiv analysieren zu können.

Die technische Aufgabe war demnach auch, eine Methode und geeignete Mittel zu entwickeln, die es ermöglicht, den Metabolismus bzw. Biomolekülumsatz in Zellen des Blutes zu unterbrechen, um einen temporären, hochstabilisierten Erhalt des Ist-Zustandes von Genom, Transkriptom und Proteom zu gewährleisten und einen unkontrollierten Abbau der Moleküle zu verhindern, dabei gleichzeitig die Zellen morphologisch intakt zu halten und die Koagulation des Blutes zu verhindern. Dabei soll die Lösung der Aufgabe eine zeitversetzte Aufarbeitung für die Isolierung von CTCs und die Durchführung molekularer und ggf. morphologischer Analysen der CTCs oder anderer Zellen ermöglichen.

### Zusammenfassung der Erfindung

Die genannten Aufgaben werden durch die vorliegende Erfindung gelöst. Ein erster Aspekt der vorliegenden Erfindung ist eine Lösung zur Asservierung von Zellen, insbesondere von Tumorzellen in Blutproben oder anderen Körperflüssigkeiten, welche dadurch gekennzeichnet ist, dass sie einen membrangängigen Protonencarrier enthält und frei ist von chaotropen Stoffen.

Ferner liegt ein weiterer Aspekt der vorliegenden Erfindung in der Verwendung einer solchen Lösung gemäß dem ersten Aspekt der Erfindung zur Konservierung, Asservierung oder/und Fixierung von Zellen, insbesondere von Tumorzellen in einer Blutprobe oder einer anderen Körperflüssigkeit unter weitgehendem Erhalt der Zellmorphologie.

Ein dritter Aspekt der vorliegenden Erfindung betrifft Proberöhrchen oder Blutentnahmeröhrchen, welche eine Lösung nach dem ersten Aspekt der Erfindung enthalten.

Vorteile und Details der vorliegenden Erfindung ergeben sich aus den Ansprüchen, der folgenden detaillierten Beschreibung und den Ausführungsbespielen.

### Detaillierte Beschreibung der Erfindung

Intakte Zellmembranen sind für einen passiven Protonentransport nicht permeabel und der Protonentransport über die Zellmembran ist hoch kontrolliert. Die vorliegende Erfindung beruht auf der Erkenntnis, dass eine abrupte und schnelle Änderung (Senkung) des pH-Werts innerhalb der Zellen zu einem Funktionsverlust der zellulären Maschinen für Synthese und Abbau von Molekülen und über die Inaktivierung der enzymatischen Aktivität zu einem Stopp des Stoffwechsels führt, ohne in die zelluläre Kompartimentierung einzugreifen. Das wesentliche Kennzeichen der erfindungsgemäßen Lösung ist daher, dass die Stilllegung der Stoffwechselvorgänge sehr schnell, durch Reduktion des pH-Werts erfolgt und so die umgebungsabhängige Änderung des Expressionszustandes vermieden wird.

Des Weiteren erfolgt im Rahmen der vorliegenden Erfindung eine weitgehende Ladungsneutralisierung von Nukleinsäuren. Dies führt insbesondere bei RNA zu einer partiellen Ausfällung aus dem wässrigen Milieu und so zu einer Stabilisierung sowohl gegen enzymatische, als auch gegen alkalische Hydrolyse.

Erfindungsgemäß wird diese Änderung des intrazellulären pH durch in der Lösung enthaltene membrangängige Protonencarrier erreicht. Unter einem Protonencarrier wird ein Molekül verstanden, das in der Lage ist, unter geeigneten Bedingungen in protonierter Form die Zellmembran, bevorzugt passiv, zu überwinden und welches innerhalb der Zelle in Anion und Proton zerfällt. Dabei werden z.B. geeignete Bedingung geschaffen, indem man Zellen mit einen Protonencarrier mit einem pKs im sauren Bereich in einem sauren Milieu mischt. Hierbei passiert die protonierte Form des Protonencarriers die Zellmembran und dissoziiert aufgrund des höheren intrazellulären pHs innerhalb der Zelle. Als Ion ist ein Verlassen der Zelle des Protoncarriers nicht möglich, sodass im Gleichgewicht der intrazelluläre pH durch den extrazellulären pH bestimmt wird.

Wesentlich ist im Rahmen der vorliegenden Erfindung und insbesondere für den Erhalt der Morphologie der Zellen darüber hinaus die Abwesenheit von chaotropen Substanzen, welche im Stand der Technik in vielen entsprechenden blutkonservierenden Lösungen vorhanden sind.

Vorteilhaft werden im Rahmen der vorliegenden Erfindung membrangängige Carbonsäuren als Protonencarrier eingesetzt, nämlich vor allem C₂ bis Cs-Carbonsäuren. Wie oben bereits erwähnt, ist Kennzeichen der Erfindung der schnelle Metabolismusstopp unter Erhalt der Morphologie von Zellen im Blut. Unter Zellen werden alle nicht Hämoglobin-haltigen Zellen verstanden. Dieser Metabolismusstopp wird im Folgenden auch als Fixierung bezeichnet, obwohl der Wirk-Mechanismus der vorliegenden Erfindung von den bekannten quervernetzenden, dehydrierenden oder denaturierenden Fixierungen verschieden ist. Erreicht wird der Metabolismusstopp bevorzugt durch eine gelöste Carbonsäure in Kombination mit einem niedrigen pH. Dabei passiert die protonierte Form der Carbonsäure die Zellmembran und dissoziiert im Zellinneren. Darüber hinaus können allerdings auch andere, in protonierter Form membrangängige Säuren eingesetzt werden.

Die Säuren liegen in der erfindungsgemäßen Lösung bevorzugt in Form eines Carbonsäure-Puffersystems bzw. eines Säure/Basen-Systems vor, welches einen pH im sauren Bereich ausbildet und stabilisiert. Blut weist eine sehr hohe Pufferkapazität auf. Erfindungsgemäß wird diese Pufferkapazität mit dem in der Lösung enthaltenen Puffersystem austitriert, um in der Mischung und insbesondere innerhalb der Zellen ein saures Milieu zu schaffen, das wiederum metabolische Aktivitäten weitgehend stilllegt. Erfindungsgemäß ist ein Puffersystem geeignet, das eine ausreichende Menge protonierter Carbonsäure zur Membranpassage zur Verfügung stellt und gleichzeitig intrazellulär genügend H+ Ionen freisetzt.

Insbesondere erfolgt die Auswahl des Protonencarriers und die Einstellung des pH-Werts der erfindungsgemäßen Lösung in solcher Weise, dass sich nach der Vermengung mit der Blutprobe bzw. der anderen Körperflüssigkeit eine Mischung mit einem pH unterhalb 7 ergibt. Die entsprechende Einstellung kann der Fachmann leicht treffen, da ihm der pH-Wert und die Pufferkapazität von Blutproben oder anderen Körperflüssigkeiten, die als Probe in Frage kommen, bekannt ist und er daher je nach gewünschtem Mischungsverhältnis den pH-Wert der Lösung voreinstellen kann. Übliche Mischungsverhältnisse von zu analysierenden Blutproben und Konservierungslösungen sind dem Fach ebenfalls bekannt und betragen beispielsweise 1:10 bei Zitratblut.

In Rahmen der Erfindung wird als Carbonsäure insbesondere Essigsäure eingesetzt. Essigsäure liegt dann in der erfindungsgemäßen Lösung bevorzugt als Essigsäure/Azetat-System vor und der pH-Wert der Lösung wird so eingestellt, dass er jedenfalls unterhalb von 7 liegt. In weiteren bevorzugten Ausführungsformen wird der pH-Wert und die Pufferkapazität der erfindungsgemäßen Lösung so gewählt, dass der resultierende pH-Wert nach dem Mischen mit der Probe unterhalb von 6,2, mehr bevorzugt unterhalb von 6,0 bzw. 5,8 liegt. Insbesondere liegt der pH-Wert der Mischung zwischen 2 und 6,2, mehr bevorzugt zwischen 4,8 und 5,8 und besonders bevorzugt zwischen 5,0 und 5,5, bzw. zwischen 5,1 und 5,3. Hierzu werden insbesondere erfindungsgemäße Lösungen in Betracht kommen, deren pH-Wert unterhalb von 6,2, bevorzugt unterhalb von 5,8 und insbesondere unterhalb von 5,3 liegt. Je nach beabsichtigtem Mischungsverhältnis und zu konservierender Körperflüssigkeit zeigen solche Lösungen erfindungsgemäß einen pH-Wert im sauren Bereich, insbesondere zwischen1 und 5 und bevorzugt zwischen 1,8 und 4,5, und, wenn z.B. zur Mischung mit Blut im Verhältnis 1:5 vorgesehen, bevorzugt zwischen 2 und 3. Die Angaben zum pH-Wert beziehen sich im Rahmen der vorliegenden Erfindung auf eine Temperatur von 20°C.

In weiteren bevorzugten Ausführungsformen der Erfindung liegt ein Essigsäure/Azetat-System in der erfindungsgemäßen Lösung in einer Konzentration vor, die 10 mM bis 300 mM, bevorzugt 25 mM bis 200 mM und besonders bevorzugt 50 mM bis 150 mM beträgt. Kennzeichen der erfindungsgemäßen Lösung ist dabei die Möglichkeit der Ausgestaltung als (Mehrfach)-Konzentrat, um Blut ohne übermäßige Verdünnung zu fixieren. Hierzu wird insbesondere eine erfindungsgemäße Lösung ins Auge gefasst, die als 2 bis 20-faches, bevorzugt 5 bis 10-faches Konzentrat bezüglich der gewünschten End-Konzentration der für die Asservierung relevanten Inhaltsstoffe in der mit Blut oder einer anderen Körperflüssigkeit vermischten Probe ausgestaltet ist, also insbesondere bezüglich der Protonencarrier und dabei wiederum insbesondere des Essigsäure/Azetat-Systems. Bevorzugt wird die erfindungsgemäße Lösung in Ihrer Konzentration so eingestellt, dass nach Mischung mit der Blutprobe oder anderen Körperflüssigkeit die Konzentration der Protonencarrier, respektive des Essigsäure/Azetatsystems 5 mM bis 300 mM, bevorzugt 20 mM bis 150 mM und besonders bevorzugt 30 mM bis 50 mM beträgt

Je nach verwendetem Protonencarrier in der erfindungsgemäßen Lösung kann es erforderlich sein, zur Einstellung des pH-Werts zusätzlich eine starke Säure einzusetzen. Geeignete starke Säuren sind insbesondere Salzsäure oder andere Mineralsäuren.

Die erfindungsgemäße Lösung kann neben den genannten oder weiteren Protonencarriern auch zusätzliche Substanzen, wie insbesondere Puffersubstanzen oder übliche Hilfsstoffe aufweisen. Insbesondere kann die erfindungsgemäße Lösung Imidazol enthalten. Imidazol weist einen leicht sauren pKs auf und kann in protonierter oder nichtprotonierter Form die Zellmembran überwinden. Imidazol kann initial den Transport von H⁺-Ionen in das Zellinnere unterstützen. Eine geeignete Konzentration von Imidazol beträgt bspw. 5 mM. Es ist dem Fachmann klar, dass auch andere Imidazolkonzentrationen, bspw. 10, 25, 75, 100 mM, in geeigneter Weise zur Verminderung des intrazellulären pH beitragen können. Weitere geeignete Protonencarrier, welche im Rahmen der Erfindung als Bestandteil der Asservierungslösung eingesetzt werden können, sind zyklische Peptide wie z. B. Valinomycin oder Nigericin. Außerdem kann die erfindungsgemäße Lösung weitere Ionen, insbesondere Cl⁻-Ionen enthalten.

Die erfindungsgemäße Lösung kann auch weitere zur Konservierung beitragende Stoffe enthalten. Beispielsweise kann die Zugabe von ß-Mercaptoethanol oder Dithiotreitol oder ähnlichen Reagenzien die Aktivität von RNasen zusätzlich reduzieren. Es ist für den Fachmann selbstverständlich, dass auch die Zugabe anderer Enzym-Inhibitoren, beispielsweise Phosphatase-Inhibitoren, den konservierenden Effekt der erfindungsgemäßen Lösung positiv beeinflussen kann.

In weiteren bevorzugten Ausgestaltungen umfasst die erfindungsgemäße Lösung zusätzlich mindestens eine Aminosäure. Insbesondere handelt es sich dabei um eine im Gewebe oder in den Zellen natürlicherweise vorkommende Aminosäure. Dabei nutzt das Puffersystem der vorliegenden Erfindung den protektiven Effekt von Aminosäuren. Dieser Effekt kommt insbesondere zum Tragen, wenn derartige Aminosäuren in einer Konzentration von insgesamt oder jeweils 0,1 M bis 1M, vorzugsweise in einer Gesamtkonzentration von 100 mM bis 200 mM enthalten sind. Bevorzugt handelt es sich bei den Aminosäuren um eine oder mehrere aus der Gruppe umfassend Glycin, Alanin, Prolin, Serin, Threonin, Glutaminsäure und als Asparaginsäure.

Eine weitere Ausgestaltung der Erfindung ist die Kombination der schnellen pH abhängigen Fixierung mit einem zusätzlich konservierenden Wirkstoff aus der Gruppe der Formaldehyd-Donatoren. Solche Wirkstoffe können beispielsweise Diazolidinyl-Harnstoff oder Imidazolidinyl-Harnstoff sein.

Bevorzugt ist die Zusammensetzung der erfindungsgemäßen Lösung außerdem so gestaltet, dass die Osmolarität 1 osmol nicht überschreitet. Bevorzugt liegt die Osmolarität der Lösung über 100 und unter 500 mosmol, bevorzugt zwischen 250 und 350 mosmol. Besonders bevorzugt ist die Lösung isoton.

In einigen Ausgestaltungen der Erfindung wird durch die Formulierung der erfindungsgemäßen Lösung eine Koagulation des Blutes nicht vollständig verhindert. Es ist dem Fachmann bekannt, dass diese Koagulationserscheinungen durch Zugabe von Komplexbildnern oder inhibierenden Antikörpern unterbunden werden kann. Die Anwesenheit solcher Komplexbildner oder Antikörper in der erfindungsgemäßen Lösung führt daher zu weiteren bevorzugten Ausführungsformen der Erfindung.

Ein weiterer Aspekt der vorliegenden Erfindung ist die Verwendung der erfindungsgemäßen Lösung zur Konservierung, Reservierung oder/und Fixierung von Zellen, insbesondere Tumorzellen in einer Blutprobe oder anderen Körperflüssigkeit. Die erfindungsgemäße Verwendung erfolgt unter weitgehendem bzw. bestmöglichem Erhalt der Zellmorphologie. Im Rahmen dieses Aspekts der Erfindung ist es besonders vorteilhaft, die Lösung bereits in einem Proberöhrchen oder Blutentnahmeröhrchen oder einem anderen geeigneten Gefäß, wie der Blutentnahmespritze vorzulegen. Dies ermöglicht insbesondere die direkte Vermischung der Körperflüssigkeit nach der Entnahme mit der erfindungsgemäßen Lösung, so dass einer Verfälschung der Analyse durch zum Beispiel enzymatische Aktivitäten entgegengewirkt werden kann. Entsprechende Proberöhrchen oder Blutentnahmeröhrchen oder auch andere Reaktionsgefäße, welche die erfindungsgemäße Lösung enthalten, stellen weitere Gegenstände der vorliegenden Erfindung dar.

Hierbei ist es wiederum besonders bevorzugt, die erfindungsgemäße Lösung in konzentrierter Form vorzulegen, um eine starke Verdünnung der Probe zu vermeiden. Alternativ zu einem Vorlegen der Lösung in Proberöhrchen oder dergleichen kann auch eine sofortige Vermischung nach der Entnahme der Blutprobe oder der Körperflüssigkeit erfolgen.

Alle Erläuterungen, die im Obigen für die erfindungsgemäße Lösung aufgeführt sind, gelten gleichfalls für die erfindungsgemäße Verwendung dieser Lösung und ebenso für die vorgefertigten Proberöhrchen oder Blutentnahmeröhrchen, welche die erfindungsgemäße Lösung enthalten.

Die erfindungsgemäße Lösung und ihre Verwendung zur Fixierung und Stabilisierung von Zellen, insbesondere CTCs, ermöglicht in besonderes effektiver Weise die nachfolgende Analyse von Molekülen im Rahmen von molekularen Untersuchungen. Ein besonderes Kennzeichen der Erfindung ist die Möglichkeit, weitgehend intakte Zellen zu konservieren und sie dann z.B. anhand eines Merkmals zu selektieren und diese Selektion zu benutzen, um sie zu isolieren und molekulare Analysen durchzuführen. Die Selektion kann über alle Eigenschaften der Zellen erfolgen, bspw. exprimierte Marker, nicht exprimierte Marker oder die Zellmorphologie wie Größe oder Form, oder Lichtbrechung. Bevorzugt werden die so selektierten und isolierten Zellen einer molekularen Analyse unterworfen. Bevorzugt bestehen solche Analysen aus Analysen des Genoms, Transkriptoms, Proteoms oder Metaboloms entweder als Ganzes, oder in Teilen oder von einzelnen Genen, Transkripten, Proteinen oder Metaboliten.

Die folgenden Beispiele erläutern in Verbindung mit den Figuren die Erfindung weiter.

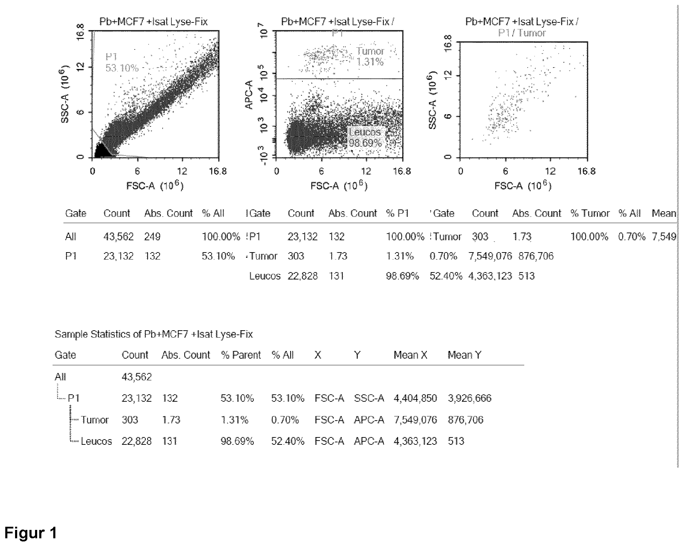

Figur 1 zeigt wie im Ausführungsbeispiel beschrieben fixiertes Blut, welches mit kultivierten Tumorzellen versetzt und nach 24 h durchflusszytometrisch untersucht wurde. Die Tumorzellpopulation ist eindeutig identifizierbar.

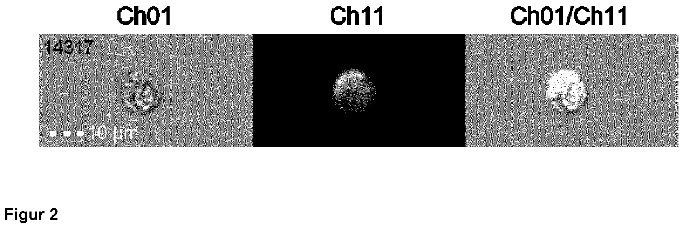

Figur 2 zeigt eine Einzelzellanalyse. Fixiertes Blut wie im Ausführungsbeispiel beschrieben wurde mit kultivierten Tumorzellen versetzt und nach 40 Stunden im Imagestream durchflusszytometrisch untersucht. Die Zellmorphologie ist erhalten.

### Beispiel

In diesem Ausführungsbeispiel wurde eine erfindungsgemäße Lösung mit folgender Zusammensetzung verwendet:
10 mM Glucose, 5 mM Imidazol, 10 mM Hepes, 3 mM Asparaginsäure, 30 mM Glutaminsäure, 5 mM Prolin, 5 mM Serin 5 mM Threonin, 5 mM Alanin 20 mM Glycin, 200 µM Natriumazid, 150 mM Essigsäure, HCl add pH 2.2

Die Lösung weist bei einer Temperatur von 20°C einen pH von 2,2 auf.

2 ml dieser Lösung wurden in eine Blutentnahmespritze vorgelegt. Die so präparierte Spritze wird verwendet, um einem Patienten 8 ml Blut abzunehmen und gleichzeitig die erfindungsgemäße Mischung herzustellen. Das so fixierte Blut kann bei 4°C bis 20°C gelagert werden, um nachfolgend, bspw. innerhalb 24 h, die Mischung auf das Vorhandensein von CTC durch mikroskopische oder durchflusszytometrische Analyse zu analysieren. Möglicherweise vorhandene CTC können auch direkt isoliert werden, bspw. über die Affinität von Oberflächenmarkern zu Antikörpern.

## Patentansprüche

1. Lösung für die Asservierung von Zellen, insbesondere von Tumorzellen in Blutproben oder anderen Körperflüssigkeiten,
**dadurch gekennzeichnet, dass** die Lösung einen zellmembrangängigen Protonencarrier enthält und frei von chaotropen Substanzen ist.

2. Lösung nach Anspruch 1,
**dadurch gekennzeichnet, dass** der Protonencarrier ausgewählt ist aus C₂-C₅ Carbonsäuren oder anderen, in protonierter Form membrangängigen Säuren und als Säure/Basen System, bevorzugt als Essigsäure/Actetat System vorliegt und einen pH im sauren Bereich ausbildet.

3. Lösung nach Anspruch 2,
**dadurch gekennzeichnet, dass** das Essigsäure/Acetatsystem in einer Konzentration von 10 mM bis 300 mM, bevorzugt 25 mM bis 200 mM und besonders bevorzugt 50 mM bis 150 mM vorliegt.

4. Lösung nach einem der vorhergehenden Ansprüche,
**dadurch gekennzeichnet, dass** sie zur Einstellung des pH zusätzlich eine starke Säure, bevorzugt Salzsäure oder eine andere Mineralsäure enthält.

5. Lösung nach einem der vorhergehenden Ansprüche,
**dadurch gekennzeichnet, dass** sie weitere Puffersubstanzen oder Protonencarrier enthält.

6. Lösung nach einem der vorhergehenden Ansprüche,
**dadurch gekennzeichnet, dass** ihr pH-Wert nach Mischung mit Zellen oder anderem biologischen Material eine pH unter 7, bevorzugt zwischen 4 und 6, und besonders bevorzugt zwischen 4,5 und 5,5 beträgt, jeweils gemessen bei 20°C.

7. Lösung nach einem der vorhergehenden Ansprüche,
**dadurch gekennzeichnet, dass** die Lösung Imidazol enthält.

8. Lösung nach einem der vorhergehenden Ansprüche,
**dadurch gekennzeichnet, dass** sie mindestens eine Aminosäure umfasst, welche bevorzugt ausgewählt ist aus der Gruppe umfassend Glycin, Alanin, Prolin, Serin, Threonin, Glutaminsäure, Asparaginsäure.

9. Lösung nach Anspruch 8,
**dadurch gekennzeichnet, dass** die eine oder mehrere Aminosäuren in einer Konzentration von insgesamt oder jeweils 0,1 M bis 1 M, vorzugsweise in einer Gesamtkonzentration von 100 mM bis 200 mM enthalten sind.

10. Lösung nach einem der vorhergehenden Ansprüche,
**dadurch gekennzeichnet, dass** sie zusätzlich einen konservierenden Wirkstoff aus der Gruppe der Formaldehyd-Donatoren, insbesondere Diazolidinyl-Harnstoff oder Imidazolidinyl-Harnstoff enthält.

11. Lösung nach einem der vorhergehenden Ansprüche,
**dadurch gekennzeichnet, dass** sie als 2 bis 20faches, bevorzugt 5 bis 10faches Konzentrat ausgestaltet ist.

12. Lösung nach einem der vorhergehenden Ansprüche,
**dadurch gekennzeichnet, dass** die Osmolarität 1 osmol nicht überschreitet, bevorzugt unter 500 mosmol liegt, und insbesondere eine isotone Umgebung für die zu asservierenden Zellen, Blutprobe oder andere Körperflüssigkeit ausbildet.

13. Lösung nach einem der vorhergehenden Ansprüche,
**dadurch gekennzeichnet, dass** sie Komplexbildner oder eine Koagulation verhindernde Antikörper enthält.

14. Verwendung einer Lösung nach einem der Ansprüche 1 bis 13 zur Konservierung, Asservierung oder/und Fixierung von Zellen, insbesondere Tumorzellen und zirkulierenden Tumorzellen, in einer Blutprobe oder anderen Körperflüssigkeit, wobei insbesondere die Lösung mit der Blutprobe oder Körperflüssigkeit direkt nach Entnahme vermischt wird oder/und die Lösung in einem Proberöhrchen oder Blutentnahmeröhrchen vorgelegt wird, wobei wiederum bevorzugt die Lösung in konzentrierter Form vorgelegt oder zugegeben wird

15. Proberöhrchen, Blutentnahmespritze oder Blutentnahmeröhrchen, welche **dadurch gekennzeichnet sind, dass** darin eine Lösung nach einem der Ansprüche 1 bis 13 enthalten ist.
